# EUROPEAN PATENT SPECIFICATION

(11) **EP 4 138 918 B1**
(45) Date of publication and mention of the grant of the patent: **22.07.2026**
(21) Application number: 21791945.5
(22) Date of filing: 21.04.2021
(51) Int. Cl.: A61K 39/42, A61K 39/395, A61K 39/44, A61K 39/02, A61K 49/16, A61K 39/12, A61K 51/10, C07K 16/08, C07K 16/10, C07K 16/42, G01N 33/543, G01N 33/569, G01N 33/58, G01N 33/96

(54) **CALIBRATION AND QUALITY CONTROL REAGENTS FOR USE WITH IMMUNOASSAYS FOR ANTIBODIES**
KALIBRIERUNGS- UND QUALITÄTSKONTROLLREAGENZIEN ZUR VERWENDUNG MIT IMMUNOASSAYS FÜR ANTIKÖRPER
RÉACTIFS D'ÉTALONNAGE ET DE CONTRÔLE DE QUALITÉ DESTINÉS À ÊTRE UTILISÉS AVEC DES DOSAGES IMMUNOLOGIQUES POUR DES ANTICORPS

(30) Priority: 24.04.2020 US 202063015223 P
(43) Date of publication of application: 01.03.2023
(60) Divisional application: 26185665.2
(73) Proprietor: Siemens Healthcare Diagnostics Inc., Tarrytown, NY 10591 (US)
(72) Inventor: BAHAR, Izak, Hockessin, Delaware 19707 (US)
(74) Representative: Schweitzer, Klaus
(86) International application number: PCT/US2021/070436
(87) International publication number: WO 2021/217178

(56) References cited:
- WO-A1-2011/143606
- WO-A1-2013/010178
- WO-A1-2019/164854
- CN-A- 109 709 317
- CN-A- 110 161 247
- US-A1- 2013 273 525
- US-A1- 2016 115 206
- ULLMAN EDWIN F ET AL: "Luminescent oxygen channeling assay (LOCITM): sensitive, broadly applicable homogeneous immunoassay method", CLINICAL CHEMISTRY, vol. 42, no. 9, 1 September 1996 (1996-09-01), US, pages 1518 - 1526, XP093110508, ISSN: 0009-9147, DOI: 10.1093/clinchem/42.9.1518
- PERERA RANAWAKA APM, CHRIS KP MOK, OWEN TY TSANG2, HUIBIN LV,RONALD LW KO , NICHOLAS C WU , MENG YUAN, WAI SHING LEUNG JACKY MC C: "Serological assays for severe acute respiratory syndrome coronavirus . 2 (SARS-CoV-2", EURO SURVEILLANCE, vol. 25, no. 16, 23 April 2020 (2020-04-23), pages 1 - 9, XP055867002, DOI: 10.2807/1560-7917. ES .2020.25.16.2000421
- HE, Y ET AL.: "Receptor-binding domain of SARS-CoV spike protein induces highly potent neutralizing antibodies: implication for developing subunit vaccine", BIOCHEMICAL AND BIOPHYSICAL RESEARCH COMMUNICATIONS, vol. 324, no. 2, 12 November 2004 (2004-11-12), pages 773 - 781, XP027154373, DOI: 10.1016/j.bbrc.2004.09.106

## Description

### BACKGROUND

In June 2020, the U.S. Food and Drug Administration (FDA) issued an Emergency Use Authorization (EUA) for a laboratory-based total antibody test developed by Siemens Healthineers (Tarrytown, NY) for the detection of the presence of SARS-CoV-2 antibodies, including IgM and IgG, in blood. A spike protein on the surface of the SARS-CoV-2 virus enables the virus to penetrate and infect human cells found in multiple organs and blood vessels. The Siemens Healthineers' Total Antibody COV2T assay was designed to detect antibodies to the spike protein. These antibodies are believed to neutralize the SARS-CoV-2 virus and therefore prevent infection. Multiple potential vaccines in development for SARS-CoV-2 include the spike protein within their focus. US 2013/273525 A1 discloses a method for simultaneously detecting total immunoglobulins and immunoglobulins M directed against this same infectious microorganism, in particular a human hepatitis virus. The method comprises placing a biological sample, in a single assay receptacle, in the presence of particles, each carrying at least one specific detectable physical parameter, and belonging to at least two different groups, one of the groups carrying an anti-lgM capture antibody and the other group carrying a capture antigen derived from said microorganism. US 2016/115206 A1 discloses a treponema pallidum triplet antigen construct which includes three treponema pallidum antigens (TP15, TP17, and TP47), as well as a ten amino acid leader sequence and human copper zinc superoxide dismutase. This construct is optimized for in vitro diagnosis of syphilis infection. CN 109 709 317 A discloses a homogeneous immunoassay kit without matrix effects. The kit includes a first composition, a second composition, a third composition and a fourth composition, wherein the first composition includes a receptor capable of reacting with singlet oxygen to generate a detectable signal and a first antibody or a first antibody fragment bound thereto. The first antibody or the first antibody fragment is capable of specifically binding with a first epitope of an analyte in a to-be-tested sample. The second composition includes a donor, wherein the donor is capable of producing singletoxygen in an excited state. The third composition includes magnet microspheres, wherein the magnet microspheres are used for separating a matrix in the to-be-tested sample from other non-specific substances that are not specifically bound with the first antibody or the first antibody fragment in the sample. The fourth composition includes a second antibody or second antibody fragment capable of specifically binding with a second epitope of the analyte or includes a known antigen capable of specifically binding with a third epitope of the analyte. Ullman Edwin F ET AL: "Luminescent oxygen channeling assay (LOCITM): sensitive, broadly applicable homogeneous immunoassay method", Clinical Chemistry, vol. 42, no. 9, 1 September 1996 (1996-09-01), pages 1518-1526, XP093110508, US ISSN: 0009-9147, DOI: 10.1093/clinchem/42.9.1518 demonstrates that LOCI assays are generally applicable to the assay of a broad range of analytes. This article concludes that the LOCI assays are homogeneous, rapid and highly robust and appear to be more sensitive than previously reported immunoassay methods. CN 110 161 247 A discloses a homogeneous immunodetection kit for detecting an anti-cyclic citrullinated peptide antibody. The kit is based on a double antigen sandwich reaction mode and prepared by adopting high sensitivity and specificity of cyclic citrullinated peptide-coupled carrier protein as an antigen raw material. The kit can detect all types of anti-citrulline antibodies and has the advantages of being strong in specificity, good in signal amplification effect, high in sensitivity, wide in linearity range, simple in operation, good in stability and high in precision.

Just as there is a need for new and improved immunoassays for COVID-19, there is a corresponding need for calibrators for these immunoassays, to ensure the accuracy thereof. In particular, anti-viral antigen antibodies are needed for the calibrators of immunoassays, such as (but not limited to) COVID-19 immunoassays. Due to the COVID-19 pandemic and a great demand for these antibodies, these anti-viral antigen antibodies have become very expensive and difficult to obtain in the quantities needed. It was forecasted that the demand for a COVID-19 immunoassay calibrator would be in the millions of vials per year, which translates into multiple grams at a cost of multi-millions of dollars. As a result, the cost of this raw material could be prohibitive and astronomical, and there are also supply issues with this raw material. As such, there is a great need to find ways to minimize the quantity of actual anti-viral antigen antibody utilized in the calibrator product.

### BRIEF DESCRIPTION OF THE DRAWINGS

FIG. 1 schematically depicts a CV2T LOCI^{®} immunoassay format using a calibration reagent containing anti-viral antigen antibody.
FIG. 2 schematically depicts a CV2T LOCI^{®} immunoassay format using a calibration reagent constructed in accordance with the present disclosure.

### DETAILED DESCRIPTION

Unless otherwise defined herein, scientific and technical terms used in connection with the present disclosure shall have the meanings that are commonly understood by those of ordinary skill in the art. Further, unless otherwise required by context, singular terms shall include pluralities and plural terms shall include the singular. The foregoing techniques and procedures are generally performed according to conventional methods well known in the art and as described in various general and more specific references that are cited and discussed throughout the present specification. The nomenclatures utilized in connection with, and the laboratory procedures and techniques of, analytical chemistry, synthetic organic chemistry, and medicinal and pharmaceutical chemistry described herein are those well-known and commonly used in the art. Standard techniques are used for chemical syntheses and chemical analyses.

All patents, published patent applications, and non-patent publications mentioned in the specification are indicative of the level of skill of those skilled in the art to which the present disclosure pertains.

All of the compositions, kits, devices, and/or methods disclosed herein can be made and executed without undue experimentation in light of the present disclosure.

As utilized in accordance with the present disclosure, the following terms, unless otherwise indicated, shall be understood to have the following meanings:

The use of the term "a" or "an" when used in conjunction with the term "comprising" in the claims and/or the specification may mean "one," but it is also consistent with the meaning of "one or more," "at least one," and "one or more than one." As such, the terms "a," "an," and "the" include plural referents unless the context clearly indicates otherwise. Thus, for example, reference to "a compound" may refer to one or more compounds, two or more compounds, three or more compounds, four or more compounds, or greater numbers of compounds. The term "plurality" refers to "two or more."

The use of the term "at least one" will be understood to include one as well as any quantity more than one, including but not limited to, 2, 3, 4, 5, 10, 15, 20, 30, 40, 50, 100, etc. The term "at least one" may extend up to 100 or 1000 or more, depending on the term to which it is attached; in addition, the quantities of 100/1000 are not to be considered limiting, as higher limits may also produce satisfactory results. In addition, the use of the term "at least one of X, Y, and Z" will be understood to include X alone, Y alone, and Z alone, as well as any combination of X, Y, and Z. The use of ordinal number terminology (i.e., "first," "second," "third," "fourth," etc.) is solely for the purpose of differentiating between two or more items and is not meant to imply any sequence or order or importance to one item over another or any order of addition, for example.

The use of the term "or" in the claims is used to mean an inclusive "and/or" unless explicitly indicated to refer to alternatives only or unless the alternatives are mutually exclusive. For example, a condition "A or B" is satisfied by any of the following: A is true (or present) and B is false (or not present), A is false (or not present) and B is true (or present), and both A and B are true (or present).

As used herein, any reference to "one embodiment," "an embodiment," "some embodiments," "one example," "for example," or "an example" means that a particular element, feature, structure, or characteristic described in connection with the embodiment is included in at least one embodiment. The appearance of the phrase "in some embodiments" or "one example" in various places in the specification is not necessarily all referring to the same embodiment, for example. Further, all references to one or more embodiments or examples are to be construed as non-limiting to the claims.

Throughout this application, the term "about" is used to indicate that a value includes the inherent variation of error for a composition/apparatus/ device, the method being employed to determine the value, or the variation that exists among the study subjects. For example, but not by way of limitation, when the term "about" is utilized, the designated value may vary by plus or minus twenty percent, or fifteen percent, or twelve percent, or eleven percent, or ten percent, or nine percent, or eight percent, or seven percent, or six percent, or five percent, or four percent, or three percent, or two percent, or one percent from the specified value, as such variations are appropriate to perform the disclosed methods and as understood by persons having ordinary skill in the art.

As used in this specification and claim(s), the words "comprising" (and any form of comprising, such as "comprise" and "comprises"), "having" (and any form of having, such as "have" and "has"), "including" (and any form of including, such as "includes" and "include"), or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AAB, BBC, AAABCCCC, CBBAAA, CABABB, and so forth. The skilled artisan will understand that typically there is no limit on the number of items or terms in any combination, unless otherwise apparent from the context.

As used herein, the term "substantially" means that the subsequently described event or circumstance completely occurs or that the subsequently described event or circumstance occurs to a great extent or degree. For example, when associated with a particular event or circumstance, the term "substantially" means that the subsequently described event or circumstance occurs at least 80% of the time, or at least 85% of the time, or at least 90% of the time, or at least 95% of the time. The term "substantially adjacent" may mean that two items are 100% adjacent to one another, or that the two items are within close proximity to one another but not 100% adjacent to one another, or that a portion of one of the two items is not 100% adjacent to the other item but is within close proximity to the other item.

As used herein, the phrases "associated with" and "coupled to" include both direct association/binding of two moieties to one another as well as indirect association/binding of two moieties to one another. Non-limiting examples of associations/couplings include covalent binding of one moiety to another moiety either by a direct bond or through a spacer group, non-covalent binding of one moiety to another moiety either directly or by means of specific binding pair members bound to the moieties, incorporation of one moiety into another moiety such as by dissolving one moiety in another moiety or by synthesis, and coating one moiety on another moiety, for example.

The terms "analog" and "derivative" are used herein interchangeably and refer to a substance which comprises the same basic carbon skeleton and carbon functionality in its structure as a given compound, but can also contain one or more substitutions thereto. The term "substitution" as used herein will be understood to refer to the replacement of at least one substituent on a compound with a residue R. In certain non-limiting embodiments, R may include H, hydroxyl, thiol, a halogenid selected from fluoride, chloride, bromide, or iodide, a C1-C4 compound selected one of the following: linear, branched or cyclic alkyl, optionally substituted, and linear branched or cyclic alkenyl, wherein the optional substitutents are selected from one or more alkenylalkyl, alkynylalkyl, cycloalkyl, cycloalkenylalkyl, arylalkyl, heteroarylalkyl, heterocyclealkyl, optionally substituted heterocycloalkenylalkyl, arylcycloalkyl, and arylheterocycloalkyl, each of which is optionally substituted wherein the optional substitutents are selected from one or more of alkenylalkyl, alkynylalkyl, cycloalkyl, cyclalkenylalkyl, arylalkyl, alkylaryl, heteroarylalkyl, heterocyclealkyl, optionally substituted heterocycloalkenylalkyl, arylcycloalkyl, and arylheterocyclalkyl, phenyl, cyano, hydroxyl, alkyl, aryl, cycloalkyl, cyano, alkoxy, alkylthio, amino, -NH (alkyl), -NH(cycloalkyl)₂, carboxy, and -C(O))-alkyl.

The term "sample" as used herein will be understood to include any type of biological sample that may be utilized in accordance with the present disclosure. Examples of fluidic biological samples that may be utilized include, but are not limited to, whole blood or any portion thereof (i.e., plasma or serum), urine, saliva, sputum, cerebrospinal fluid (CSF), skin, intestinal fluid, intraperitoneal fluid, cystic fluid, sweat, interstitial fluid, extracellular fluid, tears, mucus, bladder wash, semen, fecal, pleural fluid, nasopharyngeal fluid, combinations thereof, and the like.

The term "antibody" is used herein in the broadest sense and refers to, for example, intact monoclonal antibodies and polyclonal antibodies, multi-specific antibodies (e.g., bispecific antibodies), as well as antibody fragments and conjugates thereof that exhibit the desired biological activity of analyte binding (such as, but not limited to, Fab, Fab', F(ab')2, Fv, scFv, Fd, diabodies, single-chain antibodies, and other antibody fragments and conjugates thereof that retain at least a portion of the variable region of an intact antibody), antibody substitute proteins or peptides (i.e., engineered binding proteins/peptides), and combinations or derivatives thereof. The antibody can be of any type or class (e.g., IgG, IgE, IgM, IgD, and IgA) or sub-class (e.g., IgG1, IgG2, IgG3, IgG4, IgA1, and IgA2).

The term "LOCI^{®}" as used herein refers to a commercially used immunoassay technique based on Luminescent Oxygen Channeling Assay (LOCI^{®}) technology. The LOCI^{®} advanced chemiluminescence assay is described, for example, in U.S. Pat. No. 5,340,716 (Ullman et al.). The currently available LOCI^{®} technology has high sensitivity and uses several reagents. In particular, the LOCI^{®} assay requires that two of these reagents (referred to as a "sensibead" and a "chemibead") be held by other specific binding partner assay reagents in a manner whereby the sensibead and chemibead are in close proximity to one another to achieve a signal. Upon exposure to light at a certain wavelength, the sensibead releases singlet oxygen, and if the two beads are in close proximity, the singlet oxygen is transferred to the chemibead; this causes a chemical reaction that results in the chemibead giving off light that can be measured at a different wavelength.

Disclosed herein is a calibration or quality control reagent for a serology immunoassay for the detection of antibodies to a microorganism in a human biological sample. The reagent includes three components: (a) a matrix; (b) anti-human immunoglobulin (Ig) antibodies; and (c) human antibodies against an antigen of the microorganism. The anti-human Ig antibodies and human anti-microorganism antibodies form a complex within the reagent. The formation of these complexes increases the antibody prongs capable of binding to the antigen of the microorganism. That is, each anti-human Ig antibody can bind two human anti-microorganism antibodies at the Fc fragment thereof; these two human anti-microorganism antibodies present in the complex, which are each two pronged when not complexed, become four or multi-prong antibodies upon complexation with the anti-human Ig antibodies. As such, these complexes more efficiently cross link microorganism antigens in antigen bridging immunoassays.

Any calibration or quality control reagent matrix known in the art or otherwise contemplated herein may be utilized as long as the reagent produced therefrom can function in accordance with the present disclosure. In certain non-limiting embodiments, the matrix may have a somewhat similar formulation/composition/pH to the formulations/compositions/pH of samples that will be measured in the immunoassays. However, it may be desirable in certain non-limiting embodiments to avoid the use of an unpurified serum or plasma-based matrix, as endogenous immunoglobulin present in such a matrix may interfere with the other components of-and the complexes formed in-the calibration or quality control reagent.

Non-limiting examples of samples that may be utilized in accordance with the present disclosure (and thus upon which the formulation of a matrix may be based, in certain non-limiting embodiments) include biological samples such as, but not limited to, whole blood or any portion thereof (i.e., plasma or serum), urine, saliva, sputum, cerebrospinal fluid (CSF), skin, intestinal fluid, intraperitoneal fluid, cystic fluid, sweat, interstitial fluid, extracellular fluid, tears, mucus, bladder wash, semen, fecal, pleural fluid, nasopharyngeal fluid, and combinations thereof.

In certain non-limiting embodiments, the matrix comprises human serum or plasma that has been substantially depleted of human IgG. In certain non-limiting embodiments, the matrix comprises serum from a non-human source, such as (but not limited to) horse, bovine, goat, and the like.

The matrix comprises a buffer-based system in combination with one or more proteins.

Non-limiting examples of buffers that may be utilized in accordance with the present disclosure include phosphate (such as (but not limited to) in phosphate buffered saline (PBS) solutions or Dulbecco's Phosphate saline Buffer), Tris (such as, but not limited to, a Tris buffered saline solution), Bis-Tris, borate (such as, but not limited to, a borate buffered saline solution), carbonate, citrate, glycine, ACES, BES, DIPSO, HEPES, HEPPSO, MOPS, MOPSO, PIPES, PIPPS, TAPSO, TES, a biological buffer, and the like, as well as any combinations thereof.

Non-limiting examples of proteins that may be utilized as part of the matrix in accordance with the present disclosure include an albumin (such as, but not limited to, bovine serum albumin (BSA)), casein, non-human immunoglobulin, transferrin, and the like, as well as any combinations thereof.

Also, the matrix may contain additional components, as is well known in the art. For example (but not by way of limitation), the matrix may include one or more preservatives, one or more bulking agents, and/or one or more polymers. Non-limiting examples of bulking agents that may be utilized in accordance with the present disclosure include lactose, sorbitol, trehalose, sucrose, mannitol, glycine, and the like, as well as any combinations thereof. Non-limiting examples of preservatives that may be utilized in accordance with the present disclosure include ProClin^{™} (Rohm and Haas, Philadelphia, PA), sodium azide, thimerosal, neomycin, clotrimazole, amphotericin B, MIT, streptomycin, OMADINE^{®} (sodium pyrithione; Lonza, Basel, Switzerland), COSMOCIL^{®} CQ (Lonza, Basel, Switzerland), and the like, as well as any combinations thereof. Non-limiting examples of polymers that may be utilized in accordance with the present disclosure include polyethlene glycol (PEG), Dextran, Polyvinylpyrrolidone (PVP), and the like, as well as any combinations thereof.

In particular (but non-limiting) embodiments, the matrix comprises bovine serum albumin (BSA) in PBS. One non-limiting example of a matrix is 6% protease-free BSA in PBS, pH 7.4.

The serology immunoassay with which the calibration or quality control reagents of the present disclosure are utilized may detect human antibodies to an antigen of any microorganism for which detection is desired. For example (but not by way of limitation), the microorganism to be detected may be a bacteria, a virus, a protozoan, a fungus, or the like.

Non-limiting examples of bacteria that can be detected in accordance with the present disclosure include *Acinetobacter, Actinomyces, Aeromonas, Aggregatibacter, Atopobium, Bacillus, Bacteroides, Bartonella, Bifidobacterium, Borellia, Brucella, Campylobacter, Chlamydia, Chlamydophila, Clostridium, Corynebacterium, Coxiella, Eikenella, Enterobacter, Enterococcus, Escherichia, Eubacterium, Francisella, Fusobacterium, Gardnerella, Haemophilis, Helicobacter, Klebsiella, Lactobacillus, Listeria, Mobiluncus, Moraxella, Mycobacterium, Mycoplasma, Neisseria, Parviomonas, Pasteurella, Porphyromonas, Prevotella, Propionibacterium, Proteus, Pseudomonas, Rickettsia, Salmonella, Serratia, Shigella, Staphylococcus, Streptococcus, Tannerella, Treponema, Vibrio,* and *Yersinia* species, and the like.

Non-limiting examples of viruses that can be detected in accordance with the present disclosure include adenoviruses, astroviruses, coronaviruses, Coxsackie viruses, cytomegaloviruses (CMV), echoviruses, encephalitis viruses, enteroviruses, Epstein-Barr viruses (EBV), erythroviruses, hantaviruses, hepatitis viruses, herpes viruses, human immunodeficiency viruses (HIV), influenza viruses, noroviruses, papilloma viruses, parainfluenza viruses, paramyxoviruses, polio viruses, rabies viruses, respiratory syncytial viruses (RSV), rhinoviruses, rotoviruses, rubella viruses, rubeola viruses, Varicella-Zoster viruses, West Nile viruses, and Zika viruses, and the like.

Non-limiting examples of protozoans that can be detected in accordance with the present disclosure include *Ascaris, Babesia, Cryptosporidium, Cyclospora, Entamoeba, Enterobius, Giardia, Hymenolepis, Necator, Plasmodium, Strongyloides, Taenia, Toxoplasma,* and *Trichomonas* species, and the like.

Non-limiting examples of fungi that can be detected in accordance with the present disclosure include yeasts, molds, and the like, including (but not limited to) *Candida, Cryptococcus, Epidermophyton, Malassezia, Microsporum,* and *Trichophyton* species, and the like.

In a particular (but non-limiting) embodiment, the microorganism detected by the immunoassay is severe acute respiratory syndrome coronavirus 2 (SARS-CoV-2), HIV, Hepatitis B Core Total, Epstein-Barr virus, Herpes Virus (HSV), CMV, Rubella, *H. pylori,* or *Toxoplasma gondii.*

The antigen may be any antigen from the microorganism to be detected. Antigens that are useful in detection of each of the microorganisms listed above are well known in the art and widely available. In addition, the selection of antigens that may be utilized in accordance with the present disclosure is well within the purview of a person having ordinary skill in the art. Thus, no further disclosure thereof is deemed necessary.

Human antibodies against these antigens are also widely known and commercially available. In addition, antibody manufacturers produce an extremely large array of thousands of human anti-microorganism antibodies that can be utilized in accordance with the present disclosure; see, for example (but not by way of limitation), the antibody catalogs of manufacturers such as Roche (Basel, Switzerland), Genentech (San Francisco, CA), Aridis Pharmaceuticals LLC (San Jose, CA), Medimmune, LLC (Gaithersberg, MD), XBiotech (Austin, TX), Rockland Immunochemicals, Inc. (Pottstown, PA), USBiological Life Sciences (Swampscott, MA), Santa Cruz Biotechnology, Inc. (Dallas, TX), Jackson Immuno Research Labs, Inc. (West Grove, PA), Thermo Fisher Scientific (Waltham, MA), and Sigma-Aldrich Corp. (St. Louis, MO). However, this list is only a small sampling of antibody manufacturers that produce human anti-microorganism antibodies, and there are many additional commercial sources of human anti-microorganism antibodies that can be utilized in accordance with the present disclosure. Thus, a person having ordinary skill in the art will clearly and unambiguously be able to identify and select a variety of human anti-microorganism antibodies that can be utilized in accordance with the present disclosure, and as such, no further description of the human anti-microorganism antibodies or the characteristics thereof is deemed necessary.

In a particular (but non-limiting) embodiment, the human anti-microorganism antibody is a humanized anti-SARS-CoV-2 antibody, such as (but not limited to) the humanized anti-RBD of S1 antibody obtained from GenScript (Piscataway, NJ), and the antigen to which the human antibodies are directed is an antigen of SARS-CoV-2, such as (but not limited to) at least a portion of the receptor binding domain (RBD) of the S1 subunit of the CoV spike protein.

The anti-human Ig antibodies may specifically bind to any portion of any human immunoglobulin molecules known in the art or otherwise contemplated herein. For example (but not by way of limitation), the antibodies may be directed to human IgG, IgE, IgM, IgD, and/or IgA, and/or any portion thereof (such as, but not limited to, anti-human gamma chain, anti-human H+L, anti-human light chain, and the like). Anti-human Ig antibodies are well known in the art, are widely commercially available, and have been vastly studied. For example (but not by way of limitation), a few commercial sources of anti-human IgG monoclonal and/or polyclonal antibodies include Rockland Immunochemicals, Inc. (Pottstown, PA), USBiological Life Sciences (Swampscott, MA), Santa Cruz Biotechnology, Inc. (Dallas, TX), Jackson Immuno Research Labs, Inc. (West Grove, PA), Thermo Fisher Scientific (Waltham, MA), and Sigma-Aldrich Corp. (St. Louis, MO). However, this list is not inclusive, and there are many additional commercial sources of anti-human Ig antibodies that can be utilized in accordance with the present disclosure. Thus, a person having ordinary skill in the art will clearly and unambiguously be able to identify and select a variety of anti-human Ig antibodies that can be utilized in accordance with the present disclosure, and as such, no further description of the anti-human Ig antibodies or the characteristics thereof is deemed necessary.

Each of the anti-human immunoglobulin (Ig) antibodies and the human antibodies against a microorganism antigen may be present in the calibration or quality control reagent at any concentration that allows complexes to be formed as described herein and that allows the reagent to function in accordance with the present disclosure. In certain non-limiting embodiments, each of the anti-human immunoglobulin (Ig) antibodies and the human antibodies against a microorganism antigen is present in the reagent at a concentration independently selected from about 0.001 wt%, about 0.002 wt%, about 0.003 wt%, about 0.004 wt%, about 0.005 wt%, about 0.006 wt%, about 0.007 wt%, about 0.008 wt%, about 0.009 wt%, about 0.01 wt%, about 0.02 wt%, about 0.03 wt%, about 0.04 wt%, about 0.05 wt%, about 0.06 wt%, about 0.07 wt%, about 0.08 wt%, about 0.09 wt%, about 0.1 wt%, about 0.2 wt%, about 0.3 wt%, about 0.4 wt%, about 0.5 wt%, about 0.6 wt%, about 0.7 wt%, about 0.8 wt%, about 0.9 wt%, about 1.0 wt%, about 1.1 wt%, about 1.2 wt%, about 1.3 wt%, about 1.4 wt%, about 1.5 wt%, about 1.6 wt%, about 1.7 wt%, about 1.8 wt%, about 1.9 wt%, about 2 wt%, about 3 wt%, about 4 wt%, about 5 wt%, about 6 wt%, about 7 wt%, about 8 wt%, about 9 wt%, about 10 wt%, or higher, or a range formed of two of the above values (i.e., a range of from about 0.001 wt% to about 10 wt%, a range of from about 0.002 wt% to about 1 wt%, a range of from about 0.001 wt% to about 0.5 wt%, etc.), or any value that falls within a range of two of the above values (i.e., about 0.27 wt%).

Likewise, the human antibodies against a microorganism antigen and the anti-human immunoglobulin (Ig) antibodies may be present in the reagent at any ratio to one another that allows the reagent to function in accordance with the present disclosure. For example (but not by way of limitation), the anti-human Ig antibodies and the human anti-microorganism antibodies may be present in a ratio in a range of from about 1:1, about 1:1.5, about 1:2, about 1:2.5, about 1:3, about 1:3.5, about 1:4, about 1:4.5, about 1:5, about 1:5.5, about 1:6, about 1:6.5, about 1:7, about 1:7.5, about 1:8, about 1:8.5, about 1:9, about 1:9.5, about 1:10, about 2:3, about 2:5, about 2:7, about 2:9, about 2:11, about 2:13, about 2:15, about 2:17, about 2:19, or higher, or a range formed of two of the above values (i.e., a range of from about 1:1 to about 1:10, a range of from about 1:2 to about 2:3, etc.), or any value that falls within a range of two of the above values (i.e., about 1:1.7, about 1:2.4, etc.). When desired, the ratio can be optimized for each reagent to achieve a good balance between stability and signal. The ratio can also vary widely depending upon the particular reagents used.

Similarly, any additional components present in the matrix (i.e., one or more protein(s), preservative(s), bulking agent(s), and/or polymer(s)) may each be present at any concentration that allows the reagents formed therefrom to function in accordance with the present disclosure. For example (but not by way of limitation), each additional component may be present at a concentration independently selected from about 0.001 wt%, about 0.002 wt%, about 0.003 wt%, about 0.004 wt%, about 0.005 wt%, about 0.006 wt%, about 0.007 wt%, about 0.008 wt%, about 0.009 wt%, about 0.01 wt%, about 0.02 wt%, about 0.03 wt%, about 0.04 wt%, about 0.05 wt%, about 0.06 wt%, about 0.07 wt%, about 0.08 wt%, about 0.09 wt%, about 0.1 wt%, about 0.2 wt%, about 0.3 wt%, about 0.4 wt%, about 0.5 wt%, about 0.6 wt%, about 0.7 wt%, about 0.8 wt%, about 0.9 wt%, about 1.0 wt%, about 1.1 wt%, about 1.2 wt%, about 1.3 wt%, about 1.4 wt%, about 1.5 wt%, about 1.6 wt%, about 1.7 wt%, about 1.8 wt%, about 1.9 wt%, about 2 wt%, about 3 wt%, about 4 wt%, about 5 wt%, about 6 wt%, about 7 wt%, about 8 wt%, about 9 wt%, about 10 wt%, about 15 wt%, about 20 wt%, about 25 wt%, about 30 wt%, about 35 wt%, about 40 wt%, about 45 wt%, about 50 wt%, about 55 wt%, about 60 wt%, about 65 wt%, about 70 wt%, about 75 wt%, about 80 wt%, about 85 wt%, about 90 wt%, about 95 wt%, or higher, or a range formed of two of the above values (i.e., a range of from about 0.001 wt% to about 20 wt%, a range of from about 0.1 wt% to about 5 wt%, a range of from about 2 wt% to about 20 wt%, etc.), or any value that falls within a range of two of the above values (i.e., about 13 wt%, about 23 wt%, etc.).

The calibration or quality control reagent of the present disclosure is substantially stable at a desired temperature for a desired period of time. For example (but not by way of limitation), the calibration or quality control reagent may be substantially stable for at least about 7 days, at least about 14 days, at least about 28 days, at least about 1 month, at least about 2 months, at least about 3 months, at least about 4 months, at least about 5 months, at least about 6 months, at least about 7 months, at least about 8 months, at least about 9 months, at least about 10 months, at least about 11 months, at least about 12 months, at least about 13 months, at least about 14 months, at least about 15 months, at least about 16 months, at least about 17 months, at least about 18 months, at least about 2 years, at least about 3 years or longer. Also, the calibration reagent may be stored at room temperature (i.e., a range of from about 18°C to about 20°C), or stored under refrigerated or frozen conditions. When a preservative is present in the calibration or quality control reagent, the preservative utilized may be selected based upon the particular storage conditions and storage period desired.

The calibration or quality control reagents of the present disclosure may be provided in any form that allows the reagent to function in accordance with the present disclosure. For example, but not by way of limitation, the reagent may be provided in liquid form. Alternatively, the reagent may be freeze-dried or lyophilized and provided in the form of a dry reagent.

The calibration or quality control reagents of the present disclosure may be utilized for qualitative or quantitative measurements.

The present invention is directed to kits according to claim 1 that contain any of the calibration or quality control reagents described or otherwise contemplated herein.

The kits further include at least one reagent for use in the serology immunoassay. For example, but not by way of limitation, the kits may further include at least one reagent that contains the antigen of the microorganism to which the human anti-microorganism antibodies bind.

In a particular (but non-limiting) embodiment, the microorganism is SARS-CoV-2, and the antigen is the RBD of SARS-CoV-2 Spike S1. This antigen can be obtained from any source known in the art. For example (but not by way of limitation), this particular antigen is commercially available from GenScript (Piscataway, NJ), Meridian Life Sciences, Inc. (Memphis, TN), Sino Biological US Inc. (Wayne, PA), ACRO Biosystems (Newark, DE), Biorbyt, LLC (St. Louis, MO), Icosagen, AS (San Francisco, CA), and Bios Pacific Inc. (Emeryville, CA).

The kits further include one or more reagents for performing an immunoassay of a particular assay format, such as (but not limited to) the LOCI^{®} format. The kit further includes a composition comprising a singlet oxygen-activatable chemiluminescent compound (such as (but not limited to) a chemibead) and a composition comprising a sensitizer (such as (but not limited to) a sensibead), each having the microorganism antigen directly or indirectly attached thereto (or the kit containing reagents for directly or indirectly attaching the compositions to the microorganism antigen, either prior to or during the immunoassay).

The kit further includes: (i) a composition comprising: a singlet oxygen-activatable chemiluminescent compound having directly or indirectly bound thereto the antigen to which the human anti-microorganism antibodies of the reagent specifically bind, and a fluorescent molecule that is excited by the activated chemiluminescent compound; and (ii) a composition comprising a sensitizer capable of generating singlet oxygen in its excited state and having directly or indirectly bound thereto the antigen to which the human anti-microorganism antibodies of the reagent specifically bind. In another particular (but non-limiting) embodiment, the kit may further include: (i) a composition comprising: a singlet oxygen-activatable chemiluminescent compound having directly or indirectly bound thereto the antigen to which the human anti-microorganism antibodies of the reagent specifically bind, and a fluorescent molecule that is excited by the activated chemiluminescent compound; (ii) a composition comprising a sensitizer capable of generating singlet oxygen in its excited state and having a biotin-specific binding partner directly or indirectly bound thereto; and (iii) an antigen to which the human anti-microorganism antibodies of the reagent specifically bind, wherein the antigen is biotinylated.

A chemiluminescent compound (chemiluminescer) is a compound that is chemically activatable and, as a result of such activation, emits light at a certain wavelength. Examples of chemiluminescers, by way of illustration and not limitation, include: olefins capable of reacting with singlet oxygen or a peroxide to form hydroperoxides or dioxetanes, which can decompose to ketones or carboxylic acid derivatives; stable dioxetanes which can decompose by the action of light; acetylenes which can react with singlet oxygen to form diketones; hydrazones or hydrazides that can form azo compounds or azo carbonyls such as (but not limited to) luminol; and aromatic compounds that can form endoperoxides, for example. As a consequence of the activation reaction, the chemiluminescers directly or indirectly cause the emission of light.

In certain embodiments, the singlet oxygen-activatable chemiluminescent compound may be a substance that undergoes a chemical reaction with singlet oxygen to form a metastabile intermediate species that can decompose with the simultaneous or subsequent emission of light. The composition comprising the chemiluminescent compound may be directly excited by the activated chemiluminescent compound; alternatively, the composition may further comprise at least one fluorescent molecule that is excited by the activated chemiluminescent compound.

A sensitizer is a molecule, usually a compound, that generates a reactive intermediate such as, for example, singlet oxygen, for activation of a chemiluminescent compound. In some non-limiting embodiments, the sensitizer is a photosensitizer. Other sensitizers that can be chemi-activated (by, e.g., enzymes and metal salts) include, by way of example and not limitation, other substances and compositions that can produce singlet oxygen with or without activation by an external light source. For example, certain compounds have been shown to catalyze the conversion of hydrogen peroxide to singlet oxygen and water. Non-limiting examples of other sensitizer substances and compositions include: oxides of the alkaline earth metals Ca, Sr, and Ba; derivatives of elements of groups 3A, 4A, SA, and 6A in d⁰ configuration; oxides of actinides and lanthanides; and oxidizers ClO⁻, BrO⁻, Au³⁺, IO₃⁻, and IO₄⁻; and in particular, molybdate, peroxomolybdate, tungstate, and peroxotungstate ions, and acetonitrile. The following references, provide further disclosure regarding sensitizer substances and compositions that also fall within the scope of the present disclosure: Aubry, J. Am. Chem. Soc., 107:5844-5849 (1985); Aubry, J. Org. Chem., 54:726-728 (1989); Böhme and Brauer, Inorg. Chem., 31:3468-3471 (1992); Niu and Foote, Inorg. Chem., 31:3472-3476 (1992); Nardello et al., Inorg. Chem., 34:4950-4957 (1995); Aubry and Bouttemy, J. Am. Chem. Soc., 119:5286-5294 (1997); and Almeida et al., Anal. Chim. Acta, 482:99-104 (2003).

Also included within the scope of photosensitizers are compounds that are not true sensitizers but which on excitation by heat, light, ionizing radiation, or chemical activation will release a molecule of singlet oxygen. Members of this class of compounds include, for example (but not by way of limitation), the endoperoxides such as 1,4-biscarboxyethyl-1,4-naphthalene endoperoxide; 9,10-diphenylanthracene-9,10-endoperoxide; and 5,6,11,12-tetraphenyl naphthalene 5,12-endoperoxide. Heating or direct absorption of light by these compounds releases singlet oxygen.

A photosensitizer is a sensitizer for activation of a photoactive compound, for example, by generation of singlet oxygen by excitation with light. The photosensitizers are photoactivatable and include, e.g., dyes and aromatic compounds, and are usually compounds comprised of covalently bonded atoms, usually with multiple conjugated double or triple bonds. The compounds should absorb light in the wavelength range of from about 200 nm to about 1,100 nm, such as (but not limited to) a range of from about 300 nm to about 1,000 nm or a range of from about 450 nm to 950 nm, with an extinction coefficient at its absorbance maximum greater than 500 M⁻¹ cm⁻¹, or greater than 5,000 M⁻¹ cm⁻¹, or greater than 50,000 M⁻¹ cm⁻¹, at the excitation wavelength. Photosensitizers should be relatively photostable and may not react efficiently with singlet oxygen. Examples of photosensitizers, by way of illustration and not limitation, include: acetone; benzophenone; 9-thioxanthone; eosin; 9,10-dibromoanthracene; methylene blue; metallo-porphyrins such as (but not limited to) hematoporphyrin; phthalocyanines; chlorophylls; rose bengal; and buckminsterfullerene; as well as derivatives of these compounds.

Particular, non-limiting examples of chemiluminescent compounds and photosensitizers that may be utilized in accordance with the present disclosure are set forth in U.S. Pat. No. 5,340,716 (Ullman, et al.).

Any biotin-specific binding partners known in the art or otherwise contemplated herein may be utilized in accordance with the present disclosure. In certain non-limiting embodiments, the biotin-specific binding partner is an antibody against biotin. In other non-limiting embodiments, the biotin-specific binding partner is avidin or an analog thereof.

Any avidin analogs known in the art or otherwise contemplated herein may be utilized in accordance with the present disclosure, so long as the avidin or avidin analog is: (1) capable of association with the sensitizer; (2) capable of binding to the biotinylated analyte-specific binding partner; and (3) capable of binding to biotin that may be present in a sample. Non-limiting examples of avidin analogs that can be utilized in accordance with the present disclosure include those disclosed in Kang et al. (J Drug Target (1995) 3:159-65). Particular non-limiting examples of avidin analogs include avidin, streptavidin, traptavidin, neutral avidin, Neutralite avidin, Neutravidin, Lite-avidin, succinylated avidin, other forms of modified or genetically engineered) avidin, esters, salts, and/or derivatives of any of the above, and the like.

Any fluorescent molecules known in the art that are capable of being excited by the activated chemiluminescent compound and emitting light at a particular, detectable wavelength can be utilized in accordance with the present disclosure as the fluorescent molecules of (a) and (b) (as well as (e), if present), so long as the signals produced by each fluorescent molecule is detectable from the signals produced by the other fluorescent molecules utilized. That is, the fluorescent molecule of (a) must emit light at a wavelength that is sufficiently different from the wavelength at which the fluorescent molecule of (b) emits light so that the two signals can be distinguished from one another when detected simultaneously. In a particular (but non-limiting) example, each fluorescent molecule utilized in accordance with the present disclosure is independently selected from the group consisting of terbium, uranium, samarium, europium, gadolinium, and dysprosium. For example (but not by way of limitation), terbium emits light at a wavelength of about 545 nm, uranium emits light at a wavelength of about 612 nm, and samarium emits light at a wavelength of about 645 nm.

The assay components/reagents present in the kits may be provided in any form that allows them to function in accordance with the present disclosure. For example but not by way of limitation, each of the reagents may be provided in liquid form and disposed in bulk and/or single aliquot form within the kit. Alternatively, in a particular (but non-limiting) embodiment, one or more of the reagents may be disposed in the kit in the form of a single aliquot lyophilized reagent. The use of dried reagents in microfluidics devices is described in detail in US Patent No. 9,244,085 (Samproni).

In addition to the assay components/reagents described in detail herein above, the kits may further contain other reagent(s) for conducting any of the particular assays described or otherwise contemplated herein. The nature of these additional reagent(s) will depend upon the particular assay format, and identification thereof is well within the skill of one of ordinary skill in the art; therefore, no further description thereof is deemed necessary. Also, the components/reagents present in the kits may each be in separate containers/compartments, or various components/reagents can be combined in one or more containers/compartments, depending on the cross-reactivity and stability of the components/reagents. In addition, the kit may include a microfluidics device in which the components/reagents are disposed.

The relative amounts of the various components/reagents in the kits can vary widely to provide for concentrations of the components/reagents that substantially optimize the reactions that need to occur during the assay methods and further to optimize substantially the sensitivity of an assay. Under appropriate circumstances, one or more of the components/reagents in the kit can be provided as a dry powder, such as a lyophilized powder, and the kit may further include excipient(s) for dissolution of the dried reagents; in this manner, a reagent solution having the appropriate concentrations for performing a method or assay in accordance with the present disclosure can be obtained from these components. Non-limiting examples of other reagents that can be included in the kits include wash solutions, dilution solutions, excipients, interference solutions, positive controls, negative controls, and the like. In addition, the kit can further include a set of written instructions explaining how to use the kit. A kit of this nature can be used in any of the methods described or otherwise contemplated herein.

Disclosed herein is a method of producing any of the calibration or quality control reagents for a serology immunoassay for antibodies to a microorganism in a human biological sample. In the method, human antibodies against an antigen of the microorganism are combined with anti-human Ig antibodies in a matrix, and incubated under conditions whereby a complex is formed between an anti-human Ig antibody and one or more of the human antibodies against an antigen of the microorganism.

The incubation step may be performed at any temperature and for any period of time that allows for the formation of one or more of the complexes described herein. For example, but not by way of limitation, the incubation step may be performed at a temperature in a range of from about 10°C to about 50°C (such as, but not limited to, a range of from about 18°C to about 40°C, and in particular (but not by way of limitation), about 37°C), and for a period of time of about 30 seconds, about 60 seconds, about 2 minutes, about 3 minutes, about 4 minutes, about 5 minutes, about 10 minutes, about 15 minutes, about 20 minutes, about 25 minutes, about 30 minutes, about 35 minutes, about 40 minutes, about 45 minutes, about 50 minutes, about 55 minutes, about 1 hour, about 1.5 hours, about 2 hours, about 2.5 hours, about 3 hours, about 4 hours, about 5 hours, about 6 hours, about 7 hours, about 8 hours, about 12 hours, about 18 hours, about 24 hours, or longer, as well as a range formed of any two of the above values (i.e., a range of from about 1 minute to about 5 hours, a range of from about 30 minutes to about 2 hours, etc.), or a value that falls between two of the above values (i.e., about 53 minutes, about 15 hours, etc.).

In addition, the method of producing any of the calibration or quality control reagents may contain one or more additional steps in order to form the reagents described herein.

Any human anti-microorganism antibody capable of detection via the assay formats described or otherwise contemplated herein may be detected by the immunoassays of the present disclosure. In a particular (but non-limiting) embodiment, the antibody is an anti-viral antigen antibody for the SARS-Covid2 virus.

Signal producing system (sps) members are utilized that comprise a sensitizer such as, for example, a photosensitizer, and a chemiluminescent-fluorescent molecule composition, and each of which have the antigen of the microorganism directly or indirectly attached thereto (or are capable of having the antigen directly or indirectly attached thereto during the assay); in these assay embodiments, activation of the sensitizer results in a product that activates the chemiluminescent composition(s), thereby generating a detectable signal that relates to the amount of bound human anti-microorganism antibody being detected. An exemplary (but non-limiting) embodiment of an assay platform on which the present disclosure can be based is the Luminescence Oxygen Channeling Assay (LOCI^{®}; Siemens Healthcare Diagnostics Inc., Tarrytown, NY). The LOCI^{®} assay is described, for example, in U.S. Pat. No. 5,340,716 (Ullman et al.).

The assay is conducted by incubating the calibration or quality control reagent with two or more reagents for of the LOCI^{®} format. For example (but not by way of limitation), the calibration or quality control reagent may be incubated with a singlet oxygen-activatable chemiluminescent compound (such as (but not limited to) a chemibead) and a composition comprising a sensitizer (such as (but not limited to) a sensibead), each having the antigen of the microorganism directly or indirectly bound thereto. In a particular (but non-limiting) embodiment, the calibration or quality control reagent is simultaneously or partially or wholly sequentially combined with: (i) a composition comprising: a singlet oxygen-activatable chemiluminescent compound having directly or indirectly bound thereto the antigen to which the human anti-microorganism antibodies of the reagent specifically bind, and a fluorescent molecule that is excited by the activated chemiluminescent compound; and (ii) a composition comprising a sensitizer capable of generating singlet oxygen in its excited state and having directly or indirectly bound thereto the antigen to which the human anti-microorganism antibodies of the reagent specifically bind. In another particular (but non-limiting) embodiment, the calibration or quality control reagent is simultaneously or partially or wholly sequentially combined with: (i) a composition comprising: a singlet oxygen-activatable chemiluminescent compound having directly or indirectly bound thereto the antigen to which the human anti-microorganism antibodies of the reagent specifically bind, and a fluorescent molecule that is excited by the activated chemiluminescent compound; (ii) a composition comprising a sensitizer capable of generating singlet oxygen in its excited state and having a biotin-specific binding partner directly or indirectly bound thereto; and (iii) a biotinylated antigen to which the human anti-microorganism antibodies of the reagent specifically bind.

In the second step, the components are incubated together to allow for the binding of the chemiluminescent compound-containing composition and the sensitizer-containing composition to the human anti-microorganism antibodies present in the complexes of the calibration or quality control reagent, thereby resulting in the formation of a complex/aggregate in which the sensitizer is brought into close proximity to the chemiluminescent compound.

In the third step, the sensitizer is activated to generate singlet oxygen, wherein activation of the sensitizer present in the complex/aggregate causes the activation of the chemiluminescent compound present in each complex/aggregate.

In the fourth step, the amount of chemiluminescence generated by the activated chemiluminescent compound in the complex/aggregate is determined by measuring the amount of light emitted by the fluorescent molecule to determine the amount of human anti-microorganism antibodies in the calibration or quality control reagent.

For example, in certain particular (but non-limiting) embodiments, the singlet oxygen-activatable chemiluminescent compound is a substance that undergoes a chemical reaction with singlet oxygen to form a metastabile intermediate species that can decompose with the simultaneous or subsequent emission of light.

In particular (but non-limiting) embodiments, the sensitizer is a photosensitizer, and the activation of the sensitizer in step (3) comprises irradiation with light (such as, but not limited to, irradiation at about 680 nm).

As mentioned above, the various components of the method are provided in combination (either simultaneously or sequentially). When the various components of the method are added sequentially, the order of addition of the components may be varied; a person having ordinary skill in the art can determine the particular desired order of addition of the different components to the assay. The simplest order of addition, of course, is to add all the materials simultaneously and determine the signals produced therefrom. Alternatively, each of the components, or groups of components, can be combined sequentially. In certain embodiments, an incubation step may be involved subsequent to one or more additions. While particular embodiments of the present disclosure are described as having the LOCI^{®} assay format, it is to be understood that the present disclosure is also directed to any other immunoassay formats (and kits, microfluidics devices, and methods of performing same) that are capable of detecting human antibodies to microorganism antigens.

### EXAMPLE

An Example is provided hereinbelow. However, the present disclosure is to be understood to not be limited in its application to the specific experimentation, results, and laboratory procedures disclosed herein. Rather, the Example is simply provided as one of various embodiments and is simply meant to be exemplary, not exhaustive.

In this Example, anti-human IgG antibodies were used to complex anti-viral antigen (AVAg) antibodies in a calibrator base matrix. The complexed AVAg antibodies, which are two pronged when not complexed, become four or multi-prong antibodies upon complexation with the anti-human IgG antibodies. Such a complex (which contains two or more AVAg antibodies complexed to an anti-human Ig antibody) can more efficiently crosslink the viral antigens in antigen bridging assays.

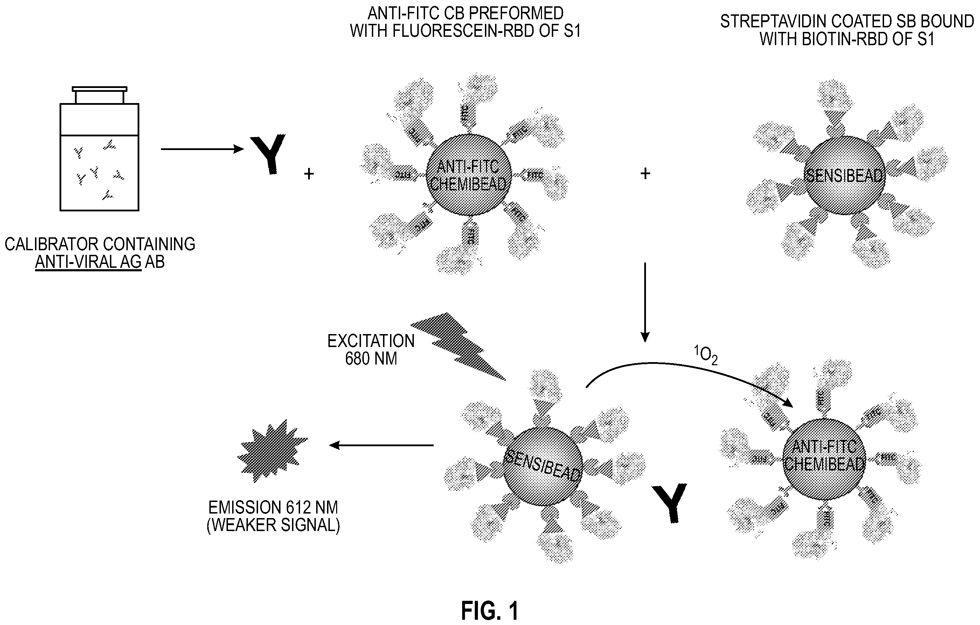

For example, as shown in FIG. 1, a calibrator that simply contains AVAg antibody binds in a 1:1 ratio to a chemibead and a sensibead in a CV2T LOCI^{®} assay format. In particular, an anti-FITC chemibead preformed with fluorescein-labeled receptor-binding domain (RBD) of the S1 subunit of SARS-CoV-2 spike protein was mixed with streptavidin-coated sensibead bound with biotinylated RBD of S1 and the calibrator containing AVAg antibody. For a signal to be generated, one prong of the AVAg antibody must bind to a chemibead, and the other prong of the same AVAg antibody must bind to a sensibead. Following incubation of the chemibead and sensibead with the calibrator, the mixture is subjected to excitation at 680 nm, which causes the sensibead to release singlet oxygen. The close proximity of chemibead and sensibead (via binding to the same antibody) causes the singlet oxygen to be transferred to the chemibead, and this causes a chemical reaction that results in the chemibead giving off an emission of light that can be measured at 612 nm.

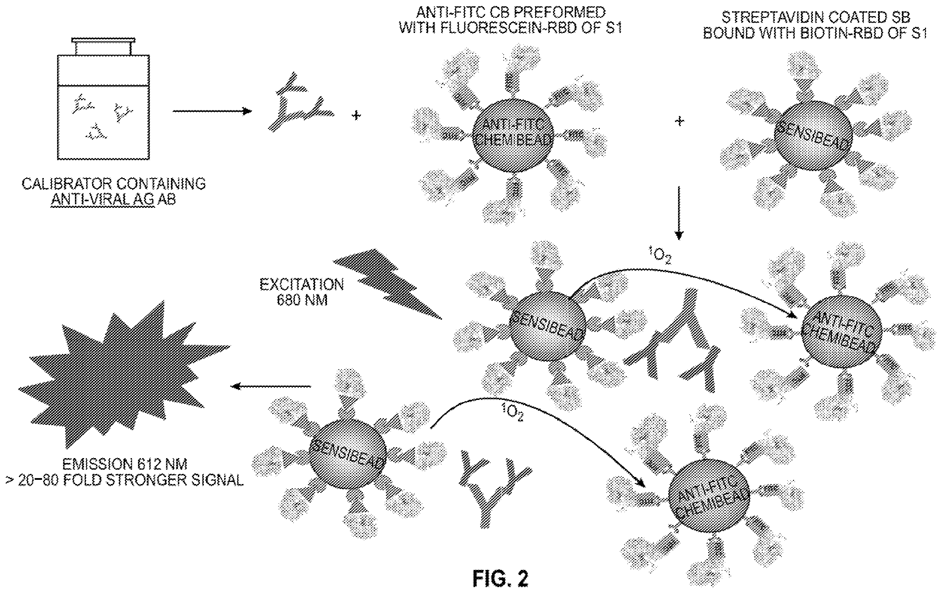

In contrast, as shown in FIG. 2, a calibrator produced in accordance with the present disclosure was utilized so that a complex of AVAg antibodies bound to anti-human IgG antibody were formed. These complexes present in the calibrator can contain two AVAg antibodies bound to each anti-human IgG antibody, thus providing an agent within the calibrator that contains at least four prongs that can each bind to the anti-viral antigen (i.e., in the instance of the CV2T assay, the RBD of S1). When this calibrator was incubated with the chemibead and sensibead from the CV2T LOCI^{®} assay, each complex that contained four prongs could bind up to four antigens, each present on either a chemibead or sensibead, and thereby allowed for the formation of aggregates that contained two or more chemibeads and/or two or more sensibeads. The formation of these aggregates greatly enhanced the chances of a sensibead being in close enough proximity to a chemibead at the time of exposure to the excitation wavelength, and thus exponentially enhanced the emission of light given off by the various chemibeads present in the aggregates upon exposure to the excitation wavelength. As such, the signal generated by the calibration reagent of the present disclosure can produce a signal that is about 20- to about 80-fold stronger (or more) than the signal generated by the calibrator utilized in FIG. 1.

The calibrator was produced as follows: Due to the abundant endogenous antibodies in human serum/plasma, BSA was selected as the calibrator matrix (although other materials/samples that are free of human serum and plasma may also be used). In 6% protease-free BSA in PBS, pH 7.4 buffer as a base matrix, humanized chimeric anti-RBD Ig antibodies (GenScript, Piscataway, NJ) were mixed with one of two different anti-human monoclonal antibody clones: 4H5 (Siemens Healthcare Diagnostics, Tarrytown, NY) and 2F3 (Thermo Fisher Scientific, Waltham, MA), at a ratio of about 1:2 anti-human Ig antibody versus AVAg antibody. This mixture was incubated at about 37°C for at least one hour prior to storage.

The monoclonal antibody 4H5 was generated by immunizing mice with human IgG, and this monoclonal antibody specifically binds to the constant domain of the IgG heavy chain. For production, the hybridoma cell line was grown in cell culture, and the culture supernatant was purified using Protein A affinity chromatography. This monoclonal antibody has shown specificity in terms of binding exclusively to human IgG

In a particular sample amplification study, the calibration reagents produced above were utilized in a CV2T LOCI^{®} assay containing the chemibead and sensibead of FIGS. 1-2. As shown in Table 1, each of the two calibration reagents (which contained complexes formed with multi-pronged AVAg antibodies) enhanced the assay signal by about 20 to 25 times that seen when the calibration reagent contained non-complexed AVAg antibodies, in this viral antigen bridging immunoassay.

**TABLE 1**

| **Calibrator** | **Sample** | **Signal** | **Mean** |
|---|---|---|---|
| No Antibody | Buffer | 11.6 | 11.3 |
| | | 11.2 | |
| | | 10.9 | |
| Anti-viral Antigen Antibody | Buffer | 48.8 | 57.1 |
| | | 65.8 | |
| | 5 µg/ml anti-RBD of S1 | 56.8 | |
| Anti-viral Ag complexed with anti-human Ig Ab clone 4H5 | Buffer | 1318.1 | 1336.7 |
| | 5 µg/ml anti-RBD of S1 | 1424.0 | |
| | 2.5 µg/ml 4H5 | 1268.0 | |
| Anti-viral Ag complexed with anti-human Ig Ab clone 2F3 | Buffer | 1097.2 | 1107.7 |
| | 5 µg/ml anti-RBD of S1 | 1140.9 | |
| | 2.5 µg/ml 2F3 | 1085.1 | |

With this high level of signal enhancement, the quantity of anti-viral antigen antibody needed for producing a calibration or quality control reagent is greatly reduced, thereby resulting in at least a 10- to 20-fold cost savings.

Thus, in accordance with the present disclosure, there have been provided kits, which fully satisfy the objectives and advantages set forth hereinabove

## Claims

1. A kit comprising:
at least one calibration or quality control reagent for a serology immunoassay for detection of antibodies to a microorganism in a human biological sample, the reagent comprising:
(a) a matrix comprising a buffer-based system in combination with one or more proteins;
(b) anti-human immunoglobulin (Ig) antibodies; and
(c) human antibodies against an antigen of the microorganism; and wherein a complex of (b) and (c) is formed in the reagent,
at least one reagent for use in the serology immunoassay, and
(i) a composition comprising:
a singlet oxygen-activatable chemiluminescent compound having directly or indirectly bound thereto the antigen to which the human antibodies of (c) of the reagent specifically bind; and
a fluorescent molecule that is excited by the activated chemiluminescent compound; and
either
(ii) a composition comprising a sensitizer capable of generating singlet oxygen in its excited state and having directly or indirectly bound thereto the antigen to which the human antibodies of (c) of the reagent specifically bind; or
(ii) a composition comprising a sensitizer capable of generating singlet oxygen in its excited state and having a biotin-specific binding partner directly or indirectly bound thereto; and
(iii) a biotinylated antigen to which the human antibodies of (c) of the reagent specifically bind.

2. The kit of claim 1, wherein the matrix comprises BSA in PBS.

3. The kit of claim 1, wherein the matrix comprises at least one additional component selected from a polymer, a preservative, a bulking agent, and combinations thereof.

4. The kit of claim 1, wherein the microorganism is a virus, wherein the virus is severe acute respiratory syndrome coronavirus 2 (SARS-CoV-2), preferably wherein the antigen of SARS-CoV-2 to which the antibody of (c) is directed is at least a portion of the receptor binding domain (RBD) of S1 subunit of CoV spike protein.

5. The kit of claim 1, wherein (b) and (c) are present in a ratio in a range of from about 1:1 to about 1:10.

6. The kit of claim 1, wherein (b) is present in the reagent at a concentration in a range of from about 0.01 wt% to about 10 wt%.

7. The kit of claim 1, wherein (c) is present in the reagent at a concentration in a range of from about 0.01 wt% to about 10 wt%.

8. The kit of claim 1, further comprising at least one composition comprising the antigen of the microorganism, wherein the antigen comprises at least a portion of the receptor binding domain (RBD) of the CoV spike protein S1 subunit.

## Patentansprüche

1. Kit, umfassend:
mindestens ein Kalibrations- oder Qualitätskontrollreagenz für einen Serologie-Immunassay zum Nachweis von Antikörpern gegen einen Mikroorganismus in einer menschlichen biologischen Probe, wobei das Reagenz Folgendes umfasst:
(a) eine Matrix, die ein pufferbasiertes System in Kombination mit einem oder mehreren Proteinen umfasst;
(b) Anti-menschliches-Immunglobulin(Ig)-Antikörper und
(c) menschliche Antikörper gegen ein Antigen des Mikroorganismus und,
wobei ein Komplex aus (b) und (c) in dem Reagenz gebildet wird, mindestens ein Reagenz zur Verwendung bei dem Serologie-Immunassay und
(i) eine Zusammensetzung, die Folgendes umfasst:
eine Singulett-Sauerstoff-aktivierbare Chemilumineszens-Verbindung, an die das Antigen, an das die menschlichen Antikörper aus (c) des Reagenzes spezifisch binden, direkt oder indirekt gebunden ist, und
ein Fluoreszenzmolekül, das durch die aktivierte Chemilumineszens-Verbindung angeregt wird, und entweder
(ii) eine Zusammensetzung, die einen Sensibilisator umfasst, der in der Lage ist, Singulett-Sauerstoff in seinem angeregten Zustand zu erzeugen, und an den das Antigen, an das die menschlichen Antikörper aus (c) des Reagenzes spezifisch binden, direkt oder indirekt gebunden ist, oder
(ii) eine Zusammensetzung, die einen Sensibilisator umfasst, der in der Lage ist, Singulett-Sauerstoff in seinem angeregten Zustand zu erzeugen, und an den ein biotinspezifischer Bindungspartner direkt oder indirekt gebunden ist, und
(iii) ein biotinyliertes Antigen, an das die menschlichen Antikörper aus (c) des Reagenzes spezifisch binden.

2. Kit nach Anspruch 1, wobei die Matrix BSA in PBS umfasst.

3. Kit nach Anspruch 1, wobei die Matrix mindestens eine zusätzliche Komponente umfasst, die aus einem Polymer, einem Konservierungsmittel, einem Streckmittel und Kombinationen davon ausgewählt ist.

4. Kit nach Anspruch 1, wobei es sich bei dem Mikroorganismus um ein Virus handelt, wobei es sich bei dem Virus um SARS-CoV-2 (severe acute respiratory syndrome coronavirus 2) handelt, wobei das Antigen von SARS-CoV-2, auf das der Antikörper aus (c) ausgerichtet ist, vorzugsweise mindestens einen Teil der Rezeptorbindungsdomäne (RBD) der S1-Untereinheit des CoV-Spike-Proteins ausmacht.

5. Kit nach Anspruch 1, wobei (b) und (c) in einem Verhältnis in einem Bereich von etwa 1:1 bis etwa 1:10 vorliegen.

6. Kit nach Anspruch 1, wobei (b) in dem Reagenz in einer Konzentration in einem Bereich von etwa 0,01 Gew.-% bis etwa 10 Gew.-% vorliegt.

7. Kit nach Anspruch 1, wobei (c) in dem Reagenz in einer Konzentration in einem Bereich von etwa 0,01 Gew.-% bis etwa 10 Gew.-% vorliegt.

8. Kit nach Anspruch 1, ferner umfassend mindestens eine Zusammensetzung, die das Antigen des Mikroorganismus umfasst, wobei das Antigen mindestens einen Teil der Rezeptorbindungsdomäne (RBD) der CoV-Spike-Protein-S1-Untereinheit umfasst.

## Revendications

1. Kit comprenant :
au moins un réactif d'étalonnage ou de contrôle qualité pour un dosage immunologique sérologique pour la détection d'anticorps contre un microorganisme dans un échantillon biologique humain, le réactif comprenant :
(a) une matrice comprenant un système à base de tampon en combinaison avec une ou plusieurs protéines ;
(b) des anticorps anti-immunoglobuline (Ig) humaine ; et
(c) des anticorps humains contre un antigène du micro-organisme ; et
dans lequel un complexe de (b) et (c) est formé dans le réactif, au moins un réactif destiné à être utilisé dans le dosage immunologique sérologique, et
(i) une composition comprenant :
un composé chimioluminescent activable par l'oxygène singulet ayant directement ou indirectement lié à celui-ci l'antigène auquel les anticorps humains de (c) du réactif se lient spécifiquement ; et
une molécule fluorescente qui est excitée par le composé chimioluminescent activé ; et soit
(ii) une composition comprenant un agent sensibilisant capable de générer de l'oxygène singulet dans son état excité et ayant directement ou indirectement lié à celui-ci l'antigène auquel les anticorps humains de (c) du réactif se lient spécifiquement ; soit
(ii) une composition comprenant un agent sensibilisant capable de générer de l'oxygène singulet dans son état excité et ayant un partenaire de liaison spécifique de la biotine lié directement ou indirectement à celui-ci ; et
(iii) un antigène biotinylé auquel les anticorps humains de (c) du réactif se lient spécifiquement.

2. Kit selon la revendication 1, dans lequel la matrice comprend de la BSA dans du PBS.

3. Kit selon la revendication 1, dans lequel la matrice comprend au moins un composant supplémentaire choisi parmi un polymère, un conservateur, un agent de charge et des combinaisons de ceux-ci.

4. Kit selon la revendication 1, dans lequel le micro-organisme est un virus, dans lequel le virus est le coronavirus 2 du syndrome respiratoire aigu sévère (SARS-CoV-2), de préférence dans lequel l'antigène du SARS-CoV-2 contre lequel l'anticorps de (c) est dirigé est au moins une partie du domaine de liaison au récepteur (RBD) de la sous-unité S1 de la protéine de pointe CoV.

5. Kit selon la revendication 1, dans lequel (b) et (c) sont présents dans un rapport dans une plage d'environ 1:1 à environ 1:10.

6. Kit selon la revendication 1, dans lequel (b) est présent dans le réactif à une concentration dans une plage d'environ 0,01 % en poids à environ 10 % en poids.

7. Kit selon la revendication 1, dans lequel (c) est présent dans le réactif à une concentration dans une plage d'environ 0,01 % en poids à environ 10 % en poids.

8. Kit selon la revendication 1, comprenant en outre au moins une composition comprenant l'antigène du microorganisme, dans lequel l'antigène comprend au moins une partie du domaine de liaison au récepteur (RBD) de la sous-unité S1 de la protéine de pointe CoV.
